# EUROPEAN PATENT APPLICATION

(11) **EP 4 464 175 A1**
(43) Date of publication of application: **20.11.2024**
(21) Application number: 23740417.3
(22) Date of filing: 09.01.2023
(51) Int. Cl.: A23L 33/18, A23L 13/20, A23L 29/00, A23P 10/30, A23P 10/22, A23P 10/28, A61K 38/01, A61K 38/07, A61K 38/08, A61P 25/28

(54) **COMPOSITION FOR IMPROVING MEMORY OR COGNITION OR PREVENTING OR TREATING CRANIAL NERVE DISEASES, COMPRISING PORCINE BRAIN HYDROLYSATES**

(30) Priority: 11.01.2022 KR 20220004195; 01.11.2022 KR 20220143631
(71) Applicant: Unimed Pharmaceuticals Inc., Asan-si, Chungcheongnam-do 31501 (KR)
(72) Inventor: KIM, Keun-Nam, Seoul 05567 (KR); SHIN, Jae-Joon, Seoul 05240 (KR); KIM, Kyung-Min, Suwon-si, Gyeonggi-do 16697 (KR); KIM, Min-Ju, Yongin-si, Gyeonggi-do 16929 (KR); HWANG, Yun-Mi, Seoul 02256 (KR); YOON, Sun-Myung, Seoul 08218 (KR); KIM, Dae-Eun, Seoul 05606 (KR); YANG, Jin-Wook, Seoul 02598 (KR); BAE, Gun-Won, Uiwang-si, Gyeonggi-do 16003 (KR)
(74) Representative: Dehns
(86) International application number: PCT/KR2023/000392
(87) International publication number: WO 2023/136573

(57) **Abstract**

The present invention relates to a composition for improving memory, improving cognition, preventing or treating cerebral neurological disease, comprising porcine brain enzyme hydrolysate. The composition can exhibit neuroprotective activity, brain-derived neurotrophic factor (BDNF) production activity, acetylcholinesterase inhibitory activity and reactive oxygen species (ROS) inhibitory activity.

## Description

### [Technical field]

This application claims priority to Korean Patent Application Serial No. 10-2022-0004195, filed Jan. 11, 2022, and Korean Patent Application Serial No. 10-2022-0143631, filed Nov. 1, 2022, the entire contents of which are hereby incorporated by reference in their entirety.

The present invention relates to a composition for improving memory, improving cognition, or preventing or treating cerebral neurological disease (herein, also refer to "brain neurological disease") comprising porcine brain enzyme hydrolysate.

### [Background art]

The brain has many functions, but the most important ones are memory and cognition. Without them, humans would have a hard time performing everyday tasks and would have trouble surviving.

Memory and cognitive impairments are the first and most common symptom experienced by people with Alzheimer's disease. In the early stages of Alzheimer's disease, patients suffer from recent memory impairment, which is the inability to recall details of recent conversations or events, due to damage to nerve cells in the hippocampus, which are less able to store recent memories. At this stage, however, remote long-term memory, the memory of events in the distant past, is relatively well preserved. As the disease progresses, however, these past memories become increasingly impaired as the cerebral cortex, which is involved in storing long-term memories, is damaged.

Meanwhile, dementia, a cognitive disorder, is one of the most problematic diseases of the elderly. Dementia refers to a persistent and general decline in cognitive functions such as memory, language, spatial and temporal awareness, judgment, and abstract thinking ability due to impaired brain function, and in severe cases, it causes many problems in daily life. Dementia is a type of brain disease and is known to be caused by a variety of causes, including Alzheimer's disease and vascular dementia, as well as degenerative brain disease such as Parkinson's disease, head trauma, and infectious disease. Therefore, there is an urgent need to treat and manage dementia, which is increasing rapidly with the exponential growth of the elderly population. Dementia refers to a syndrome that involves various acquired cognitive dysfunction symptoms such as memory, language, and behavioral disorders caused by irreversible destruction of cranial nerves due to atrophy of the brain, reduction of nerve cells, and the appearance of senile plaques. Dementia is classified into Alzheimer's disease, vascular dementia, and other dementias caused by degenerative disease such as Parkinson's disease, metabolic disease such as hypothyroidism, brain tumors, or infectious disease. Currently, acetylcholinesterase (AChE) inhibitors such as donepezil, galantamine, and rivastigmine are used in clinical practice to improve dementia, but there is no effective treatment due to low efficacy and severe side effects.

### [Disclosure]

### [Technical Problem]

The present invention provides a porcine brain enzyme hydrolysate (hereinafter, also refer to "enzyme hydrolysate of porcine brain") comprising a porcine brain-derived peptide.

The present invention provides a food composition for improving memory, improving cognitive function, or preventing or improving cerebral neurological disease comprising porcine brain enzyme hydrolysate.

The present invention also provides a health functional food composition for improving memory, improving cognitive function, or preventing or improving cerebral neurological disease.

The present invention also provides a pharmaceutical composition for improving memory, improving cognitive function, or preventing or improving cerebral neurological disease.

However, the technical challenges of the present invention are not limited to those mentioned above, and other challenges not mentioned will be apparent to those skilled in the art from the following description.

### [Technical solution]

The present invention provides a porcine brain enzyme hydrolyzate comprising a peptide consisting of SEQ ID NO: 1 or SEQ ID NO: 2, or at least one of the peptides consisting of SEQ ID NO: 1 or SEQ ID NO: 2.

The present invention provides a food composition or dietary supplement composition for improving memory, improving cognitive function, or preventing or improving cerebral neurological disease comprising porcine brain enzyme hydrolysate as an active ingredient.

The present invention also provides a pharmaceutical composition for improving memory, improving cognitive function, or preventing or treating cerebral neurological disease, comprising porcine brain enzyme hydrolysate as an active ingredient.

The porcine brain enzyme hydrolysate may comprise a porcine brain-derived peptide.

The porcine brain enzymatic hydrolysate may be hydrolyzed by one or more hydrolyzing agents.

The composition may be formulated into a dosage form selected from the group consisting of tablet, capsule, powder, granule, liquids, and pill.

The porcine brain enzyme hydrolysate may have the ability to protect nerve cells and/or produce BDNF (brain-derived neurotrophic factor).

The porcine brain enzyme hydrolysate may have acetylcholinesterase inhibitory activity and/or reactive oxygen species (ROS) inhibitory activity.

The cerebral neurological disease may be selected from the group consisting of Alzheimer's disease, mild cognitive impairment, senile dementia, Parkinson's disease, Huntington's disease, Lewy Body Dementia, Frontotemporal Dementia, cerebral infarction, stroke, and epilepsy.

The present invention also provides a method of improving memory, improving cognitive function, or preventing or treating cerebral neurological disease, comprising the step of administering or taking to an individual a composition comprising a porcine brain enzyme hydrolysate comprising at least one of the peptides consisting of SEQ ID NO: 1 or SEQ ID NO: 2 as an active ingredient.

The present invention also provides a use of a composition comprising a porcine brain enzyme hydrolysate comprising at least one of the peptides consisting of SEQ ID NO: 1 or SEQ ID NO: 2 as an active ingredient for the manufacture of a medicament/food composition for improving memory, improving cognitive function, or preventing or treating cerebral neurological disease.

### [Advantageous Effects]

The peptides according to the present invention, or porcine brain enzyme hydrolyzate comprising them, have excellent effects on improving memory, improving cognitive function, preventing or treating (or improving) cerebral neurological disease, and are not toxic, and are therefore useful in a dietary supplement composition, a pharmaceutical composition, and the like.

### [Description of Drawings]

FIG. 1 is a process diagram schematically illustrating the manufacturing process of porcine brain enzyme hydrolysate according to one embodiment of the present invention.
FIG. 2 is a graph representing the results of a cytotoxicity assay for peptides according to one embodiment of the present invention.
FIGS. 3a and 3b are graphs showing the neuroprotective activity of peptides according to one embodiment of the present invention (*, p < 0.05; ** p < 0.01; ***, p < 0.001).
FIGS. 3c and 3d are graphs showing the brain-derived neurotrophic factor (BDNF) producing ability of peptides according to one embodiment of the present invention (*, p < 0.05; ** p < 0.01; ***, p < 0.001).
FIG. 3e is a graph showing β-amyloid aggregation of a peptide (brPEP-1) according to one embodiment of the present invention.
FIG. 3f is a graph representing the Reactive Oxygen Species (ROS) inhibitory activity of peptides according to one embodiment of the present invention (*, p < 0.05; ** p < 0.01; ***, p < 0.001).
FIG. 4 is a graph showing the results of a cytotoxicity evaluation of porcine brain enzyme hydrolysate according to one embodiment of the present invention.
FIG. 5 is a graph showing the neuroprotective activity of porcine brain enzyme hydrolysate according to one embodiment of the present invention (*, p < 0.05; ** p < 0.01; ***, p < 0.001).
FIG. 6 is a graph showing the brain-derived neurotrophic factor (BDNF) production activity of porcine brain enzyme hydrolysate according to one embodiment of the present invention (*, p < 0.05; ** p < 0.01; ***, p < 0.001).
FIG. 7 shows the expression characteristics of a protein (Caspase-3, ChAT) in porcine brain enzyme hydrolysate according to one embodiment of the present invention.
FIG. 8 is a graph showing the acetylcholinesterase inhibitory activity of porcine brain enzyme hydrolysate according to one embodiment of the present invention (*, p < 0.05; ** p < 0.01).
FIG. 9 is a graph showing the reactive oxygen species (ROS) inhibitory activity of porcine brain enzyme hydrolysate according to one embodiment of the present invention (*, p < 0.05; ** p < 0.01).
FIG. 10 is a graph showing the beta-aggregation inhibitory ability of porcine brain enzyme hydrolysate according to one embodiment of the present invention.
Figure 11 shows a schematic of the passive avoidance test (a) and the results of the analysis of the memory improvement effect of pig brain enzyme hydrolysate in the passive avoidance test (b) (different letters or subscripts on the bar graphs indicate significant differences between the means at p < 0.05).
FIG. 12 shows the schematic diagram of the Y maze test (a) and the results of analyzing the memory improvement effect of pig brain enzyme hydrolysate by Y maze test (b) (different letters or subscripts on the bar graphs indicate significant differences between each other at p < 0.05).
Figure 13 shows a schematic of the Novel object test (a) and the results of analyzing the memory improvement effect of pork brain enzyme hydrolysate using the Novel object test (b) (different letters or subscripts on the bar graphs indicate significant differences between each other at p < 0.05).
FIG. 14 shows a schematic of the Morris water-maze test (a) and the results of analyzing the memory improvement effect of pig brain enzyme hydrolysate using the Morris water-maze test (b) (different letters or subscripts on the bar graphs indicate significant differences between the two at p < 0.05).

### [Mode of the Invention]

The present invention will now be described in detail.

The present invention provides a porcine brain enzyme hydrolysate comprising a peptide consisting of SEQ ID NO: 1 or SEQ ID NO: 2, or at least one of the peptides consisting of SEQ ID NO: 1 or SEQ ID NO: 2.

The peptide may be used in a food composition or a pharmaceutical composition.

The peptide may be included in the porcine brain enzyme hydrolysate at a concentration of 0.1 ppm to 300 ppm. More preferably, it may be included at a concentration of 1 to 30 ppm.

The present invention also provides a composition for improving memory, improving cognitive function, preventing or treating cerebral neurological disease, or improving cognitive function, comprising porcine brain enzyme hydrolysate as an active ingredient.

The composition may be a food composition, a health food composition, a nutraceutical composition, a dietary supplement composition, or a pharmaceutical composition.

The porcine brain enzyme hydrolysate may comprise a porcine brain-derived peptide.

The porcine brain enzymatic hydrolysate may be hydrolyzed by one or more hydrolyzing agents.

More preferably, the present hydrolysate may be produced by primary hydrolysis and secondary hydrolysis.

The primary hydrolysis can be performed by adding the protease to the solid lysate (pig brain sludge lysate), which can be performed at 40±20°C for 1 to 20 hours. However, it is not limited thereto.

The secondary hydrolysis may be performed by adding proteolytic enzymes to the solid lysate in which the primary hydrolysis was performed, and may be performed at 40±20°C for 1 to 20 hours. However, it is not limited thereto.

The composition may be formulated into a dosage form selected from the group consisting of tablet, capsule, powder, granule, liquids, and pill.

The porcine brain enzyme hydrolysate may have neuroprotective properties and/or brain-derived neurotrophic factor (BDNF) producing properties, and may also have acetylcholinesterase inhibitory properties and/or reactive oxygen species (ROS) inhibitory properties.

The cerebral neurological disease may be selected from the group consisting of Alzheimer's disease, mild cognitive impairment, senile dementia, Parkinson's disease, Huntington's disease, Lewy Body Dementia, Frontotemporal Dementia, cerebral infarction, stroke, and epilepsy.

As used herein, "preventing" refers to any act of delaying memory or cognitive decline by administration of a composition of the invention, and "treating" and "ameliorating" refers to any act of ameliorating or beneficially altering the symptoms of memory or cognitive impairment by administration of a composition of the invention.

In the present invention, the term "health functional food" refers to a food product prepared and processed in the form of tablet, capsule, powder, granule, liquids, and pills using raw materials or ingredients that have functional properties useful to the human body. The term "functional" herein refers to obtaining effects useful for health purposes, such as regulating nutrients or physiological actions on the structure and function of the human body. The health functional food of the present invention can be prepared by methods conventionally used in the art, and can be prepared by adding raw materials and ingredients conventionally added in the art. The formulation of the dietary supplement can be any formulation recognized as a dietary supplement. The dietary supplement composition of the present invention has the advantage of not having side effects that may occur when taking liquid or powdered medications for a long period of time, is highly portable, and can be taken as a supplement to prevent or improve the relevant diseases or their symptoms.

In a food composition or dietary supplement composition according to the present invention, the porcine brain enzyme hydrolysate may comprise from 1% to 20% (by weight), but not necessarily limited to 1% to 20% (by weight) of the total weight of the composition, and the amount of the mixture of active ingredients may be suitably determined according to the respective purpose of use, such as prevention, health or treatment.

Dietary supplement formulations can be in the form of acid, granules, pills, tablets, capsules, as well as in the form of regular food or beverages.

Examples of food products to which the substance may be added include, without limitation, meat, sausages, bread, chocolate, candy, snacks, confectionery, pizza, ramen, other noodles, chewing gum, dairy products including ice cream, soups, beverages, tea, drinks, alcoholic beverages and vitamin complexes, etc., and can include all foods in the conventional sense.

Generally, in the case of a liquid phase in the preparation of a food or beverage, the active ingredient may be added in an amount of not more than 15 parts by weight, preferably not more than 10 parts by weight, per 100 parts by weight of the raw material. However, in the case of prolonged consumption for health and hygiene purposes or for health control purposes, the amounts may be below the above ranges, and also above the above ranges, since the present invention does not present any safety problems in terms of utilizing fractions from natural products.

The functional food product according to the invention may comprise, as in conventional beverages, various flavoring agents or natural carbohydrates as additional ingredients. The natural carbohydrates described above may be monosaccharides such as glucose and fructose, disaccharides such as maltose and sucrose, polysaccharides such as dextrin and cyclodextrin, sugar alcohols such as xylitol, sorbitol, erythritol, etc. As sweeteners, natural sweeteners such as thaumatin, stevia extract, or synthetic sweeteners such as saccharin, aspartame, etc. can be used. The proportion of the natural carbohydrates may be about 0.01 to 0.04 g, preferably about 0.02 to 0.03 g, per 100 mL of the beverage according to the invention.

In addition to the above, the health functional food composition according to the invention may contain various nutrients, vitamins, electrolytes, flavoring agents, coloring agents, pectic acid and its salts, alginic acid and its salts, organic acids, protective colloidal thickeners, pH regulators, stabilizers, preservatives, glycerin, alcohol, carbonation agents used in carbonated beverages. In addition, the dietary supplement composition of the present invention may contain pulp for the preparation of natural fruit juices, fruit juice beverages, and vegetable beverages. These ingredients may be used independently or in combination. The proportions of these additives are not limited, but are typically selected in the range of 0.01 to 0.1 parts by weight per 100 parts by weight of the functional food composition of the invention.

The pharmaceutical composition according to the invention does not particularly limit the content of the active ingredient, but preferably the porcine brain enzymatic hydrolysate may comprise from 5 to 30 wt% by weight of the total weight of the composition. However, it is not limited thereto. However, if the active ingredient is below the above concentration range, it may be difficult to exert the desired preventive or therapeutic effect, and if it exceeds the above concentration range, the expected change in effect may be insignificant.

The pharmaceutical composition according to the invention can be formulated and used in the form of oral dosage forms, topicals, suppositories, and sterile injectable solutions, such as pills, granules, tablets, capsules, suspensions, emulsions, syrups, aerosols, and the like, each according to conventional methods, and may include suitable carriers, excipients, or diluents conventionally used in the preparation of pharmaceutical composition for formulation.

The carriers or excipients or diluents include lactose, dextrose, sucrose, sorbitol, mannitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oils, among other compounds or mixtures.

When formulated, it can be prepared using commonly used fillers, weighting agents, binders, wetting agents, disintegrants, surfactants, and other diluents or excipients.

Solid preparations for oral administration can be prepared by mixing the above active ingredient with at least one excipient, for example starch, calcium bonate, sucrose or lactose, gelatin, etc. In addition to simple excipients, lubricants such as magnesium stearate and talc can also be used.

Liquid preparations for oral use include suspensions, solutions, emulsions, and syrups, and may include a variety of excipients, such as wetting agents, sweeteners, flavors, and preservatives, in addition to the commonly used simple diluents of water and liquid paraffin.

Formulations for parenteral administration include sterile aqueous solutions, nonaqueous solvents, suspensions, emulsions, lyophilized preparations, and suppositories. Nonaqueous solvents and suspensions include propylene glycol, polyethylene glycol, vegetable oils such as olive oil, and injectable esters such as ethylolate. As a base for suppositories, witepsol, macrogol, tween 61, cacao paper, laurin paper, glycerol gelatin, etc. can be used.

The preferred dosage of the pharmaceutical composition according to the invention depends on the patient's condition, weight, severity of the disease, drug form, route of administration and duration, but can be suitably selected by those skilled in the art. However, for desirable effects, the amount of active ingredient contained in the composition of the invention may be administered in an amount generally from 0.0001 to 2,000 mg/kg per day, preferably from 0.001 to 2,000 mg/kg per day. The dose may be administered once daily or may be divided into several doses. However, the above dosages do not limit the scope of the present invention.

The pharmaceutical composition according to the invention can be administered to mammals, such as rats, mice, livestock and humans, by various routes. Any mode of administration can be, for example, by oral, rectal or intravenous, intramuscular, subcutaneous, intrauterine, intrathecal or intracerebroventricular injection.

In short-term memory behavioral tests (Y maze test, passive avoidance test, novel substance exploration test), the porcine brain enzyme hydrolysate according to the invention was found to restore short-term memory in a concentration-dependent manner (Y maze test: a test of spatial cognition; passive avoidance test: a test of remembering and avoiding stimuli; novel substance exploration test: a test of cognitive behavior of exploring a novel substance).

The present invention will now be described in more detail with reference to the following examples. The objects, features, and advantages of the present invention will be readily understood from the following examples. The invention is not limited to the embodiments described herein, but may be embodied in other forms. The embodiments described herein are provided to fully convey the ideas of the invention to those having ordinary knowledge in the technical field to which the invention belongs. Therefore, the invention should not be limited by the following embodiments.

### Example 1. Preparation of Porcine Brain Enzymatic Hydrolysate

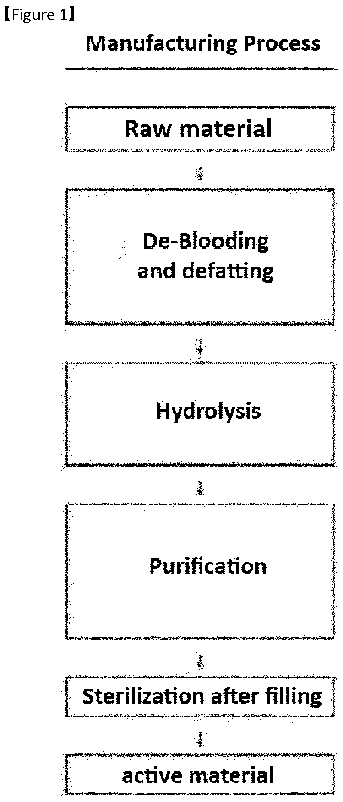

Pork brains were thawed in a thawing machine and de-blooded with fresh water to remove debris. The de-blooded pork brains were shredded using a blender to facilitate defatting. The shredded pork brains were defatted using alcohol (ethanol, isopropyl alcohol, etc.) to remove fat and debris. Defatted pig brain was subjected to primary enzymatic hydrolysis with protease (pepsin) for 1 to 20 hours. After the primary enzymatic hydrolysis was completed, filtration was performed for solid-liquid separation. After completion of filtration, protease (pancreatin) was added to the porcine brain primary hydrolysate and secondary enzymatic hydrolysis was performed for 1 to 20 hours. After the secondary enzymatic hydrolysis was completed, the proteolytic enzyme was inactivated by heating, and the pig brain enzymatic hydrolysate was contacted with a filtration aid and then filtered and adsorption purified. Afterwards, 1 to 5 times (w/w) alcohol (ethanol) was added to the filtrate of the pig brain hydrolyzate, left for 1 to 30 hours, and filtered through a filter. After concentrating the filtered liquid, the filtered liquid was divided into portions and sterilized. Finally, high purity pig brain enzyme hydrolyzate was obtained (see Figure 1).

### Example 1. Identification of peptides in porcine brain enzyme hydrolysate and analysis of peptide efficacy

### 1-1. Identification of Peptides in Porcine Brain Enzyme Hydrolysate

The porcine brain enzyme hydrolysate obtained in Example 1 was diluted by 1/2 by adding 100 µl of D.W. to 100 µl of sample. 50 µl of the above dilution was additionally diluted 1/20 by adding 950 µl of D.W. and filtered through a syringe filter and sequenced using an HPLC system on an ACQUITY UPLC I-Class PLUS (Waters) and ms/ms ionization analysis on a Xevo TQ-XS (Waters) MS system.

### <Analysis Conditions>

- Column: ACQUITY UPLC BEH C18 (2.1 × 50 mm, 1.7 µm, Waters)
- Flow rate: 200 *µ*ℓ/min
- Mobile Phase A: H2O/FA=100/0.1 (v/v)
- Mobile Phase B: Acetonitrile/FA = 100/0.1 (v/v)
- Gradient

**[Table 1]**

| Minutes (min) | A (%) | B (%) |
|---|---|---|
| 0 | 98 | 2 |
| 5 | 98 | 2 |
| 15 | 84 | 16 |
| 16 | 70 | 30 |
| 18 | 5 | 95 |
| 27 | 5 | 95 |
| 29 | 98 | 2 |
| 35 | 98 | 2 |

The results obtained by analysis under the above equipment conditions are as follows.

As a result of the analysis, a total of 4 types of peptides were analyzed, and among them, 2 types of peptides were identified as pig brain-derived peptides. The following two types that showed the most stable reproducibility were selected as indicator substances.

**[Table 2]**

| Peptide sequences of indicator substances of porcine brain enzyme hydrolysate | | | | | |
|---|---|---|---|---|---|
| No. | m/z | RT (min) | Charge | Sequence | Content in hydrolysate (ppm) |
| brPEP-1 | 403.22 | 7.6 | 1 | PSIS (SEQ ID NO: 1) | 5-30 ppm |
| brPEP-2 | 418.72 | 8.4 | 2 | GPAGPQGPR (SEQ ID NO: 2) | 5-30 ppm |

Comparison of the LC/MS EIC chromatogram (m/z 403.2) of the PSIS standard solution and the PSIS component of the porcine brain enzyme hydrolysate sample confirmed that the retention times for the PSIS peak were consistent. Furthermore, comparison of the LC/MS EIC chromatogram (m/z 418.7) of the GPAGPQGPR standard solution and the GPAGPQGPR component of the porcine brain enzyme hydrolysate sample confirmed the retention time for the GPAGPQGPR peak.

Afterwards, peptides with identical mass and MS/MS ionization forms to the peptides identified in the porcine brain hydrolysate were subsequently synthesized from Anigen (www.anygen.com) to confirm that they matched the peptides present in the porcine brain enzyme hydrolysate. Then, the following experiments were conducted.

### 1-2. Evaluate the cytotoxicity of peptides

SH-SY5Y cells, a human neuronal cell line, were obtained from the Korean Cell Line Bank (Cat No. 22266) and were cultured in Roswell Park Memorial Institute (RPMI) 1640 Medium (RPMI 1640, Cytiba) medium containing 10% fetal bovine serum (FBS, Cytiba) and 100 IU/ml penicillin and 100 µg/ml streptomycin in a 5% CO₂, 37°C incubator.

The human neuronal cell line SH-SY5Y was cultured and various concentrations of porcine brain enzyme hydrolysate-derived peptides were added to determine cell viability by MTT (methylthiazol tetrazolium bromide, Sigma Aldrich). Cells were seeded in appropriate amounts (1×10⁵ /well) in each well of a 24 well plate (BD, Falcon), treated with each concentration, and cell viability was determined after 48 hours of incubation in a 37°C incubator. After 4 hours of reaction with MTT solution, the insoluble formazan crystals were dissolved by adding 400 µl of DMSO and the absorbance was measured with an ELISA reader (TECAN, Infinite M200 pro) at a wavelength of 570 nm.

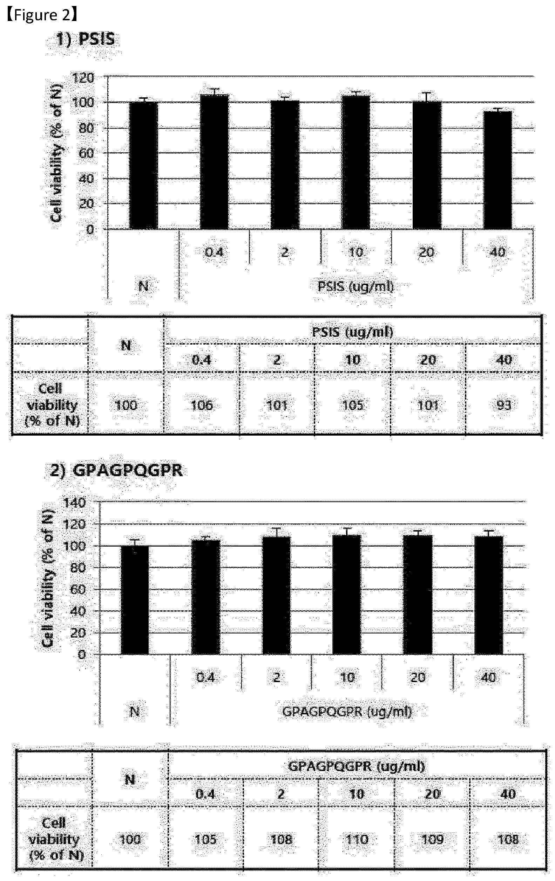

As a result, both peptides brPEP-1 and brPEP-2, which were selected as indicator substances, were found to be 93.05%±2.23% and 108.47%±5.20% at 40 ug/mL (w/v), respectively. The cell viability was more than 90% at all tested concentrations (0.4 ~ 40 ug/ml), and it was confirmed that there was no safety issue with cytotoxicity (Figure 2).

### 1-3. Evaluation of the neuroprotective effect of peptides

To evaluate the memory-enhancing efficacy of the two peptides (peptides of SEQ ID NO. 1 and SEQ ID NO. 2), human neuronal cell lines were used to determine their cytoprotective activity against neuronal cell death induced by β-amyloid treatment a non-toxic concentration or lower.

To measure neuroprotection, SH-SY5Y was toxicized by treating it with beta-amyloid, a key pathological feature of Alzheimer's disease, to induce neuronal cell death. Specifically, SH-SY5Y cells, a human neuroblastoma, were seeded at 1×10⁵ /well in 24 well plates and cultured. The cells were then pretreated with two peptides according to an embodiment of the present invention (peptide of SEQ ID NO: 1 and peptide of SEQ ID NO: 2) at different concentrations for 24 hours and incubated, and then each well was treated with beta-amyloid at a concentration of 40uM and incubated for another 24 hours. After 4 h of reaction with MTT solution (3-(4,5-dimethylthiazol-2-yl)-2,5-diphenyl tetrasodium bromide), the insoluble formazan crystals were dissolved by adding 400 µl of DMSO, and the results were confirmed by measuring the absorbance with an ELISA reader (TECAN, Infinite M200 pro) at a wavelength of 570 nm.

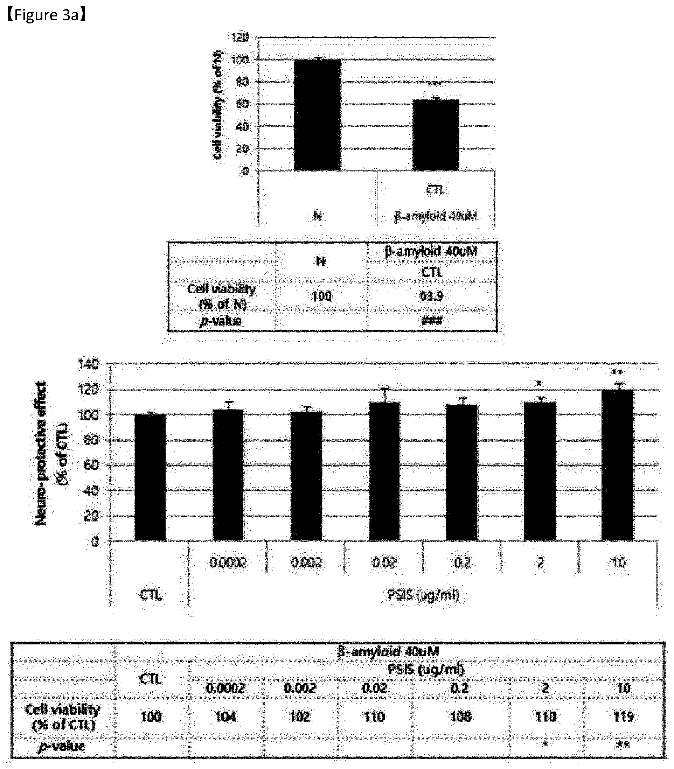

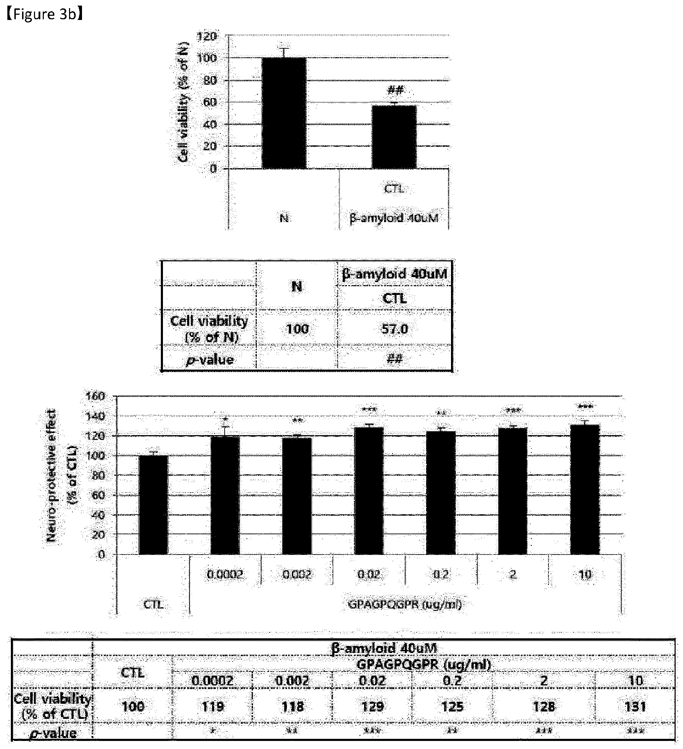

As shown in Figure 3a, cell viability was reduced to 63.7% (p < 0.001) in the Aβ25-35-treated group compared to the normal control, but pretreatment with the peptide of SEQ ID NO: 1 (PSIS) followed by treatment with 40 µM Aβ25-35 for 24 hours showed neuroprotective activity at the level of about 21-30%. In addition, as shown in Figure 3b, cell viability was reduced to 63.9% (p < 0.001) in the Aβ25-35 treated group compared to the normal control group, but pretreatment with the peptide of SEQ ID NO: 2 (GPAGPQGPR) followed by 40 µM Aβ25-35 for 24 hours showed neuroprotective activity at the level of about 18 ~ 31%.

### 1-4. Evaluate the BDNF production activity of brPEP-1 and brPEP-2

BDNF is a brain-derived neurotrophic factor. To confirm the ability of the sample to improve memory by checking the ability to produce BDNF, a protein involved in the signal transduction system of metabolic processes affecting cognitive functions (memory, thinking ability), SH-SY5Y cells, a human neuroblastoma, were cultured in 75T flasks and seeded in 6-well cell culture plates at 2×10⁵ when the confluency reached 80~90%. After one week of incubation in a 37°C, 5% CO₂ humidity chamber, the cells were treated by concentration with porcine brain enzyme hydrolysate (PBEH) according to embodiments of the present invention. After 48 hours of incubation at 37°C, 5% CO₂ humidity chamber, cell lysates were obtained and BDNF was measured using BDNF KIT (#EK0307) from Bosterbio. Afterwards, the cell number was corrected by protein content by performing a protein assay.

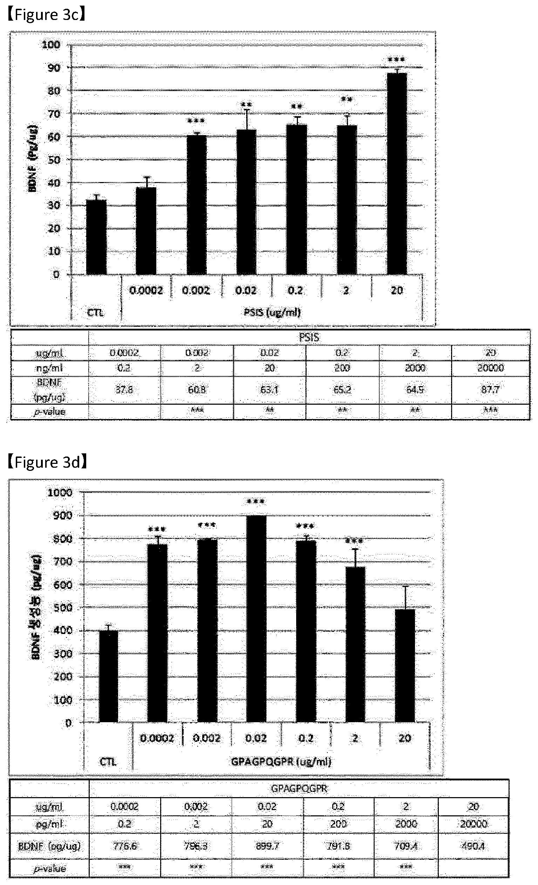

The results showed that both the peptide with SEQ ID NO: 1 (PSIS) and the peptide with SEQ ID NO: 2 (GPAGPQGPR) were found to have superior ability to produce the neurotrophic factor BDNF (Figure 3c, Figure 3d).

### 1-5. Confirmation of PEP-1 β-amyloid aggregation

Thioflavin T Assays were performed to determine the extent to which brPEP-1 according to embodiments of the present invention can inhibit the aggregation of β-amyloid. Thioflavin T shows fluorescence in response to β-amyloid. After culturing SH-SY5Y cells, each was treated with brPEP-1, and 24 hours later, treated with 20uM of beta-amyloid for 24 hours. Before harvesting the cells, they were washed with PBS (pH 7.4) and treated with 1mL of Thioflavin T 5uM. Then, it was measured at 450 nm excitation and 482 nm emission wavelengths using an ELISA reader (TECAN, Infinite M200 pro).

As Thioflavin T bound to the amyloid aggregates, the level of aggregation could be determined by the intensity of the Thioflavin T-specific fluorescence spectrum. When checking Thioflavin T over time, it was confirmed that the intensity of fluorescence was higher in the β-amyloid-treated group (CTL) than in the untreated group (N).

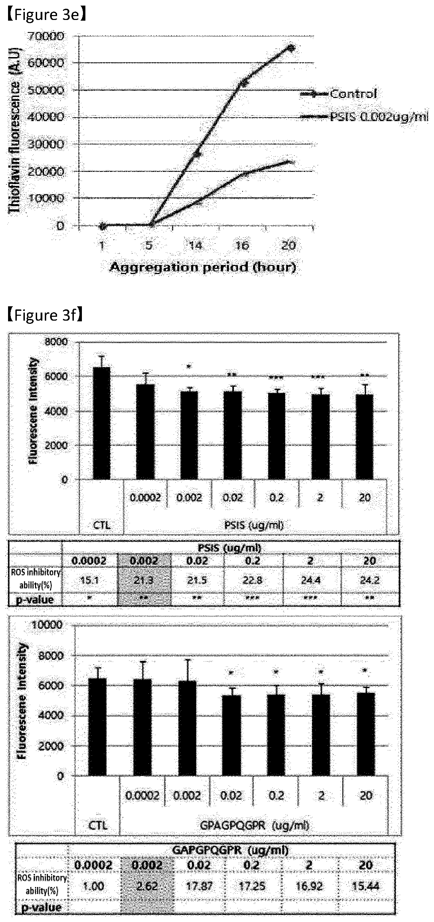

It was confirmed that brPEP-1 exhibited approximately 62% beta-amyloid aggregation inhibition after 18 hours at 0.002 ug/ml, which was the concentration present in 25 ug/ml of total nitrogen in the porcine brain enzyme hydrolysate, and that the present product exhibited similar beta-amyloid aggregation inhibition at approximately 65% after 18 hours (FIG. 3e).

### 1-6. Confirmation of reactive oxygen species (ROS) inhibition of brPEP-1 and brPEP-2

To measure the production of intracellular reactive oxygen species by H₂O₂, the fluorescent DCF-DA (2',7'-dichlorodihydrofluorescein diacetate) was used to measure intracellular reactive oxygen species levels. DCF-DA is converted to DCF in cells by reactive oxygen species and fluoresces. To measure the amount of intracellular reactive oxygen species, SH-SY5Y cells were cultured and treated with brPEP-1, brPEP-2 according to embodiments of the present invention, followed by 24 hours of treatment with H₂O₂ 100uM for 24 hours. After culturing and before harvesting the cells, they were treated with 20µM DCF-DA, incubated at 37°C for 30 minutes, washed with PBS (pH 7.4), treated with 1% triton X-100 to lysate the cells, and then measured at 480 nm excitation and 530 nm emission wavelengths using an ELISA reader (TECAN, Infinite M200 pro). brPEP-1 and brPEP-2 showed 24% and 17% inhibition at 2 ug/ml, respectively, confirming a concentration-dependent effect (Fig. 3f).

### Example 2. Analysis of efficacy of the present hydrolysate containing porcine brain-derived peptide(s)

The porcine brain enzyme hydrolysate prepared in Example 1 (including the peptides of SEQ ID NO: 1 and SEQ ID NO: 2) was analyzed for memory improvement efficacy. The content of the above peptides in the porcine brain enzymatic hydrolysate was 5 to 30 ppm, respectively.

### 2-1. Evaluation of Cytotoxicity

SH-SY5Y cells, a human neuroblastoma, were obtained from the Korean Cell Line Bank (Cat No. 22266) and were cultured in Roswell Park Memorial Institute (RPMI) 1640 Medium (RPMI 1640, Cytiba) medium containing 10% fetal bovine serum (FBS, Cytiba), 100 IU/ml penicillin and 100 µg/ml streptomycin in a 5% CO₂, 37°C incubator.

SH-SY5Y (human neuroblastoma) cells were cultured and various concentrations of porcine brain enzyme hydrolysate (PBEH) were added, and then MTT (methylthiazol tetrazolium bromide, Sigma Aldrich) was performed to determine the cell viability. Cells were seeded in appropriate amounts (1×10⁵ /well) in each well of a 24 well plate (BD, Falcon), treated with each concentration, and the cell viability was determined after 48 hours of incubation in a 37°C incubator. After 4 hours of reaction with MTT solution, the insoluble formazan crystals were dissolved by adding 400 µl of DMSO and the absorbance was measured with an ELISA reader (TECAN, Infinite M200 pro) at a wavelength of 570 nm.

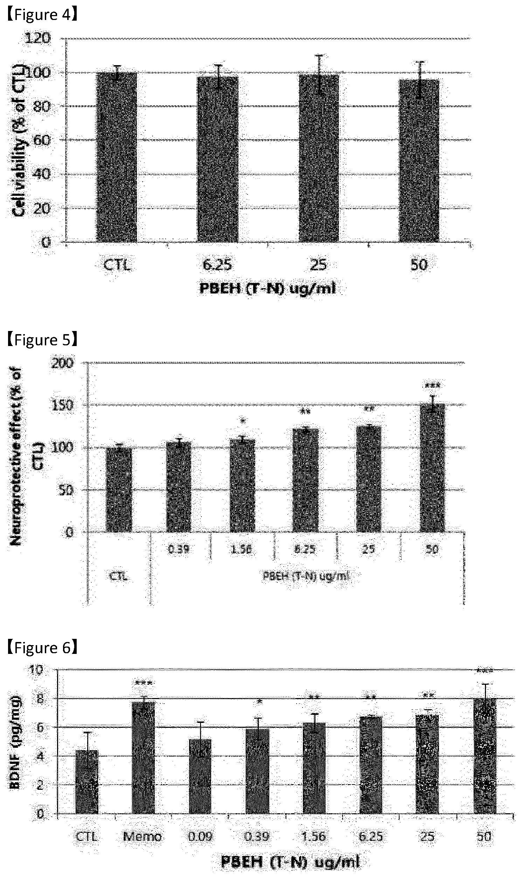

As a result, all samples of porcine brain enzymatic hydrolysate (PBEH) according to embodiments of the present invention were found to be non-toxic up to 50 ug/ml total nitrogen (FIG. 4).

### 2-2. Neuro-protective effect

To measure neuroprotection, SH-SY5Y was toxicized by treating it with beta-amyloid, a key pathological feature of Alzheimer's disease, to induce neuronal cell death. Specifically, SH-SY5Y cells, a human neuroblastoma, were seeded at 1×10⁵ /well in 24 well plates and cultured. The cells were then incubated with porcine brain enzyme hydrolysate (PBEH) according to an embodiment of the present invention for 24 hours prior to treatment by concentration, and then each well was treated with beta-amyloid at a concentration of 40uM and incubated for another 24 hours. After 4 hours of reaction with MTT solution (3-(4,5-dimethylthiazol-2-yl)-2,5-diphenyl tetrasodium bromide), the insoluble formazan crystals were dissolved by adding 400 µl of DMSO to dissolve them, and the results were confirmed by measuring the absorbance with an ELISA reader (TECAN, Infinite M200 pro) at a wavelength of 570 nm.

The results confirmed that porcine brain enzymatic hydrolysate (PBEH) according to embodiments of the present invention exhibited neuroprotective activity of 25% and 52% at 25 ug/ml and 50 ug/ml of total nitrogen (FIG. 5).

### 2-3. Ability to produce BDNF

BDNF is a brain-derived neurotrophic factor. To confirm the ability of the sample to improve memory by checking the ability to produce BDNF, a protein involved in the signal transduction system of metabolic processes affecting cognitive functions (memory, thinking ability), SH-SY5Y cells, a human neuroblastoma, were cultured in 75T flasks and seeded in 6-well cell culture plates at 2×10⁵ when the confluency reached 80~90%. After one week of incubation in a 37°C, 5% CO₂ humidity chamber, the cells were treated by concentration with porcine brain enzyme hydrolysate (PBEH) according to embodiments of the present invention. After 48 hours of incubation at 37°C, 5% CO₂ humidity chamber, cell lysates were obtained and BDNF was measured using BDNF KIT (#EK0307) from Bosterbio. Afterwards, the cell number was corrected by protein content by performing a protein assay.

The results confirmed that porcine brain enzyme hydrolysate (PBEH) exhibited 30%, 41%, and 47% BDNF production activity at 6.25, 25, and 50 ug/ml total nitrogen, respectively (Figure 6).

### 2-4. Protein expression (Caspase-3, ChAT)

In the process of apoptosis, various proteins and signal transmitters are involved, and the activation of caspase-3 is promoted, which causes the cleavage of PARP, resulting in apoptosis. To determine whether the treatment of the pig brain enzyme hydrolysate according to an embodiment of the present invention, and Ginkgo biloba leaf extract, an active ingredient for health functional food as a control, can inhibit the death of β-amyloid by reducing the activation of caspase-3, the following tests were conducted.

When the SH-SY5Y cells reached 90% confluency about 7 days after seeding, they were incubated with the above porcine brain enzyme hydrolysate (PBEH) for 24 hours and then treated with 20uM of beta-amyloid, washed with cold PBS, and lysates were obtained with RIPA buffer. Protein assay was used to determine the concentration of proteins, and then an equal amount of cell lysate and 2×sample buffer were mixed and heated at 100°C for 5 min to induce denaturation of proteins, followed by 10% SDS-polyacrylamide gel electrophoresis (SDS-PAGE). The proteins in the gel after electrophoresis were transferred to a polyvinylidene difluoride (PVDF) membrane (Bio-Rad, USA) and reacted with blocking buffer (5% skim milk) for 1 h at room temperature. Antibodies against Caspase-3, ChAT, and GAPDH were diluted 1:1,000 in Tris-buffered saline (TBS-T) containing 0.1% (v/v) tween-20 and reacted with polyvinylidene difluoride overnight at 4°C. The secondary antibody anti-rabbit IgG conjugated HRP for each antibody was diluted (1:2,000) with TBS-T, reacted at room temperature for 2 hours, and developed using an ECL kit (Bio-Rad, USA).

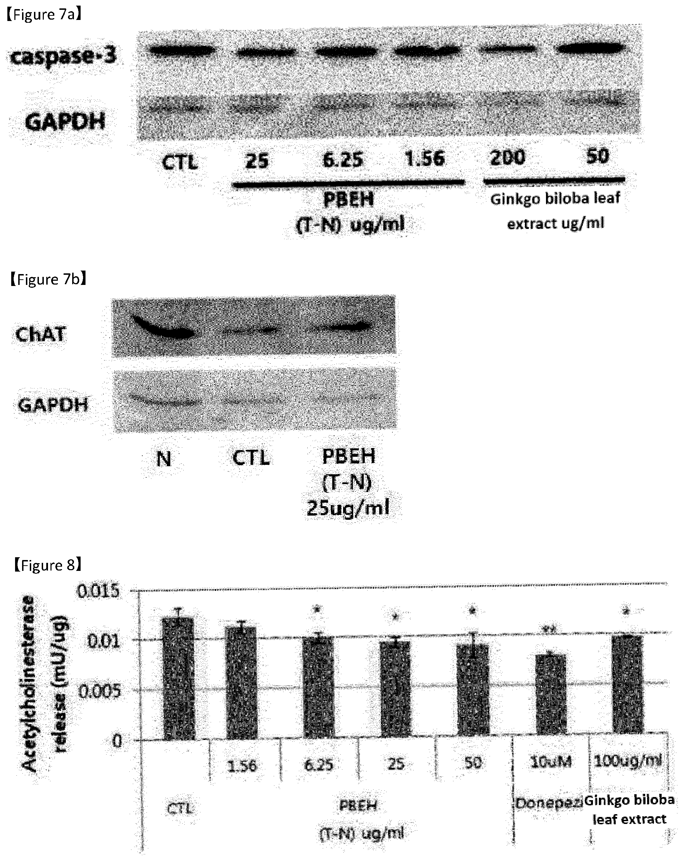

As a result of the test, it was confirmed that both Caspase-3 and ChAT had a difference in band intensity at an effective level, indicating the potential for memory improvement (Figure 7).

### 2-5. Acetylcholinesterase inhibitory activity

By inhibiting the activity of acetylcholinesterase (AChE), it is possible to increase the concentration of acetylcholine in neuronal connections by inhibiting the breakdown of acetylcholine, thereby improving cognitive function. The following test was conducted to confirm the inhibitory activity of AChE of pig brain enzyme hydrolysate.

SH-SY5Y cells, a human neuroblastoma, were cultured in 75T flasks and seeded in 6-well cell culture plates (BD, Falcon) at 2×10⁵ when confluency reached 80-90%. After one week of incubation at 37°C, 5% CO₂ humidity chamber, the cells were treated with porcine brain enzyme hydrolysate (PBEH), Ginkgo biloba leaf extract, which is currently registered as a recognized ingredient, and the drug Donepezil as a positive control. After 48 hours of incubation at 37°C, 5% CO₂ humidity chamber, cell lysates were obtained and AChE activity was measured using abcam's AChE KIT (#ab138871). Afterwards, the cell number was corrected by protein content by performing a protein assay.

The results showed that porcine brain enzyme hydrolysate (PBEH) exhibited approximately 23% and 26% inhibition at 25 and 50 ug/ml of total nitrogen, respectively, compared to approximately 21% concentration-dependent inhibition at 100 ug/ml of Ginkgo biloba leaf extract (Figure 8).

### 2-6. Reactive Oxygen Species (ROS) Inhibitory ability

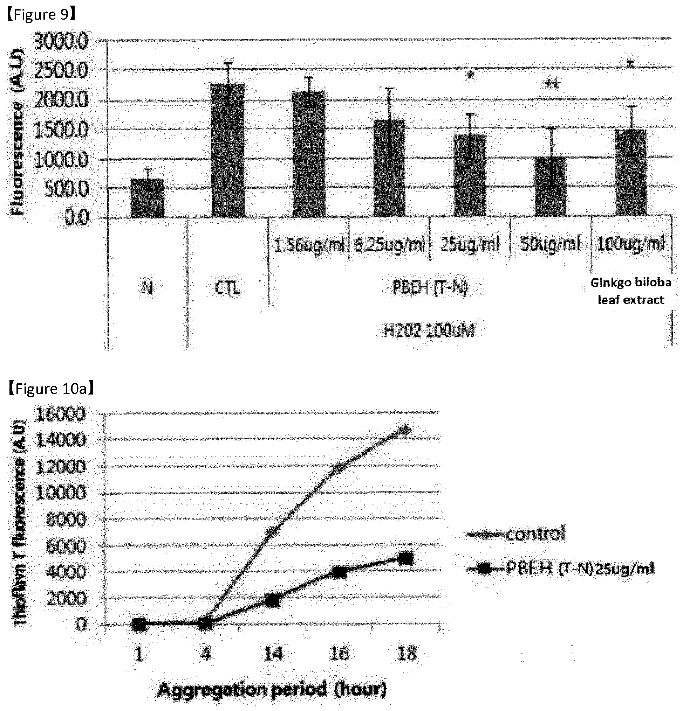

To measure the production of intracellular reactive oxygen species by H₂O₂, the fluorescent DCF-DA (2',7'-dichlorodihydrofluorescein diacetate) was used to measure intracellular reactive oxygen species levels. DCF-DA is converted to DCF in cells by reactive oxygen species and fluoresces. To measure the amount of intracellular reactive oxygen species, SH-SY5Y cells were cultured and treated with porcine brain enzyme hydrolysate (PBEH) according to an embodiment of the present invention, and ginkgo biloba leaf extract, which is currently registered as a recognized ingredient. After 24 hours, the cells were incubated with 100uM H₂O₂ for 24 hours, and then treated with 20µM DCF-DA before harvesting the cells, incubated at 37°C for 30 minutes, washed with PBS (pH 7.4), and lysed by treating the cells with 1% triton X-100. The results were then measured at 480 nm excitation and 530 nm emission wavelengths using an ELISA reader (TECAN, Infinite M200 pro). PBEH showed 43% and 60% inhibition at 25 and 50 ug/ml of total nitrogen, respectively, and confirmed that the effect was significant in a concentration-dependent manner, and at 100 ug/ml of Ginkgo biloba leaf extract showed a similar result to that of 25 ug/ml of total nitrogen with about 35.9% (Fig. 9).

### 2-7. Beta-aggregation inhibitory ability

Thioflavin T Assays were performed to determine the extent to which porcine brain enzyme hydrolysate according to embodiments of the present invention and ginkgo biloba leaf extract, which is currently registered as a recognized ingredient, could inhibit the aggregation of β-amyloid. Thioflavin T shows fluorescence in response to β-amyloid. SH-SY5Y cells were cultured and then treated with the present porcine brain enzyme hydrolysate (PBEH) and Ginkgo biloba leaf extract, followed by 24 hours of treatment with 20uM of beta-amyloid for 24 hours before cells were harvested and washed with PBS (pH 7.4) and treated with 1 mL of Thioflavin T 5uM and measured at 450 nm excitation and 482 nm emission wavelengths using an ELISA reader (TECAN, Infinite M200 pro).

As Thioflavin T bound to the amyloid aggregates, the level of aggregation could be determined by the intensity of the Thioflavin T-specific fluorescence spectrum. When checking Thioflavin T over time, it was confirmed that the intensity of fluorescence was higher in the β-amyloid-treated group (CTL) than in the untreated group (N).

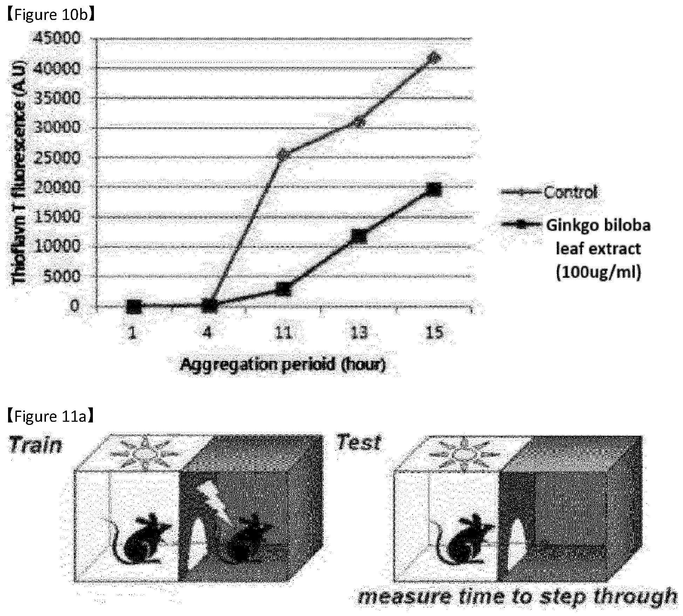

The present Porcine brain enzyme hydrolysate (PBEH) showed approximately 66% beta-amyloid aggregation inhibition at 25 ug/ml of total nitrogen, and Ginkgo biloba leaf extract was found to have approximately 46% beta-amyloid aggregation inhibition at 100 ug/ml (Figure 10).

### Example 3. Animal Behavior Tests

### 3-1. Passive avoidance test

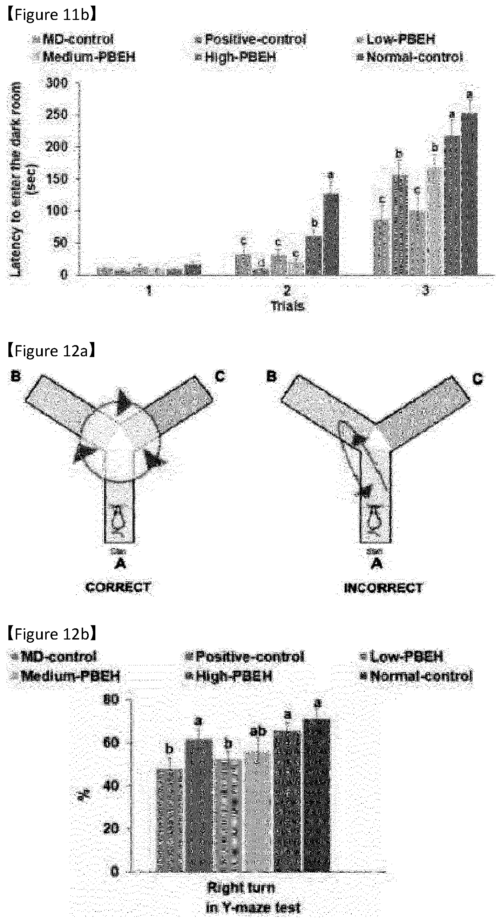

To confirm the memory/cognitive improvement effect of the porcine brain enzyme hydrolysate obtained in the above examples, a passive avoidance test was performed (FIG. 11).

### Experimental Methods

The passive avoidance test measures the time it takes for mice to move from the light room to the dark room, with the first and second tests performed 8 hours apart, and the time it takes for mice to move from the light room to the dark room after 16 hours of learning that it should not move to the dark room by electrically stimulating it when it moves to the dark room.

### Experimental results

As shown in Figure 11, in the second trial, the High-PBEH group had a long movement time similar to the normal control group (in Figure 11b, from left to right, MD-control, Positivel-control, Low-PBEH, Medium-PBEH, High-PBEH, Normal-control). In the third trial, the latency time of the normal control group was longer than that of the MD-control group (memory decline-induced group), and the Medium-PBEH (0.159 ml/kg) and High- PBEH (0.478 ml/kg) groups showed improved memory by slowing down the time to move to the dark room similar to the normal control group.

### 3-2. Y maze test

To confirm the memory/cognitive improvement effect of the porcine brain enzyme hydrolysate obtained in the above examples, a Y maze test was performed (FIG. 12). The Y maze test is a spontaneous alternating alternation and recognition memory test for the study of spatial learning and memory, which is sensitive to hippocampal damage, genetic manipulation, and memory-erasing agents and can test memory function.

### Experimental Methods

The Y-maze test is a method to evaluate short-term spatial cognition in the form of short-term memory. The Y-maze was a standardized Y-shaped blocked maze (50.5 cm long, 20 cm wide, 20 cm high, 3 arms) with three branches designated as A, B, and C. After carefully placing mice on one of the branches and letting them move freely for 8 minutes, the testers recorded the branches in which mice entered in order. One point (actual alternation) was awarded for each branch entered in turn. Alternation behavior was defined as entering all three branches without overlapping.

### Experimental results

The results showed that the number of normal turns was lower in the induced group (herein, also refer to "memory decline-induced group") compared to the normal control group. The normal turns (% of total turns) were higher in the Medium-PBEH (0.159 ml/kg) and High-PBEH (0.478 ml/kg) groups compared to the induced group(Fig. 12b, from left to right, MD-control, Positivel-control, Low-PBEH, Medium-PBEH, High-PBEH, Normal-control).

### 3-3. Novel object test

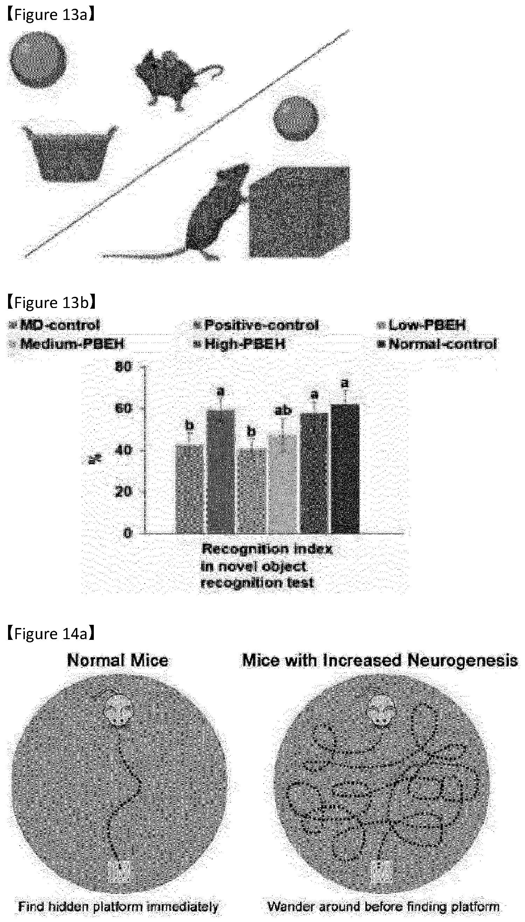

To confirm the memory/cognitive improvement effect of the porcine brain enzyme hydrolysate obtained in the above examples, a novel object test was performed (FIG. 13). The test examines the recognition of novel objects and is expressed as %, where 100% is all recognition, and the closer the value is to 100, the better the memory.

### Experimental Methods

Each mouse was placed in a plexiglass box on the first day of testing and allowed to explore a novel object for 5 min on the first day, 20 min on the second day, and 20 min on the third day. On the fourth day, the box was divided into four regions and two identical objects were placed in the center of the diagonal regions. The mouse was placed next to the box and allowed to freely explore the objects for 10 minutes. The objects and equipment were washed with 75% ethanol to remove the mouse 's odor. After 1 hour, one of the two identical objects was replaced with an object of similar size but different shape and color.

The time it took for mice to explore new and existing objects was recorded for 5 minutes. Exploratory behavior was defined as the mouse smelling and touching an object with its nose or front paws, but the mouse sitting on an object or turning around was not considered an exploratory behavior.

### Experimental results

The results showed that the induced group had lower recognition of novel objects than the normal control group, and the positive control group showed similar values to the normal control group. The pig brain enzyme hydrolysate-treated group improved the recognition of novel objects in a concentration-dependent manner, with the highest recognition rate in the High-PBEH group, confirming the improvement in cognition (Fig. 13b, from left to right, MD-control, Positive-control, Low-PBEH, Medium-PBEH, High-PBEH, Normal-control).

### 3-4. Morris water-maze test

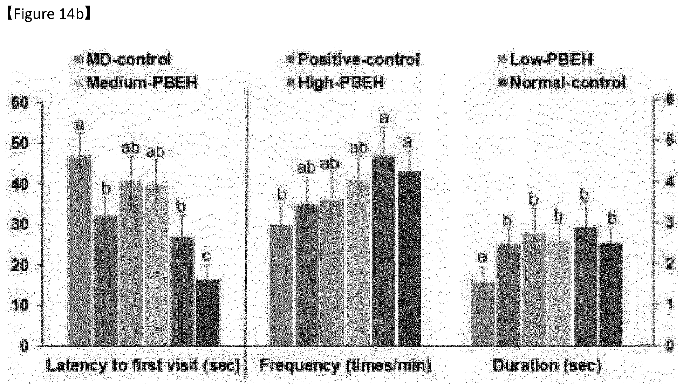

To confirm the memory/cognitive improvement effect of the porcine brain enzyme hydrolysate obtained in the above examples, a Morris water maze test was performed (FIG. 14). The Morris water maze is used to study spatial memory and learning and involves placing an animal in water and determining how long it takes to find a hidden escape platform.

### Experimental Methods

The target quadrant time, number of crossing platforms, and swimming pattern were measured and analyzed by a computer program-operated camera using the Morris water-maze test. The Morris water-maze test was performed in a circular aquarium (40 cm high × 1.5 m diameter) with a 12 cm diameter target placed on the bottom of the aquarium about 1 cm below the surface of the water, water (22°C-25°C), and skimmed milk powder to make the target invisible, and the mice were placed in zone 1 on the first day of the test, and the mice found the platform in zone 5. The water maze test was performed three times, and on days 1 and 2, the mice were trained to locate the platform in zone 5. To assess memory on day 5, the latency, frequency of visits, and duration of visits to zone 5 were measured. The test lasted 600 seconds.

### Experimental results

The results showed that the medium-PBEH (0.159 ml/kg) and high-PBEH (0.478 ml/kg) were found to have a shorter time to first discover the platform than the induced group, and the time to stay near the platform was also found to be as long as the normal control group, indicating that the porcine brain enzyme hydrolysate obtained in the present embodiment was effective for spatial memory (in FIG. 14b, from left to right, MD-control, Positive-control, Low-PBEH, Medium-PBEH, High-PBEH, Normal-control).

### 3-5. Tissue analysis of the hippocampus

To confirm the memory/cognitive improvement effect of the porcine brain enzyme hydrolysate obtained in the above examples, hippocampal tissue was analyzed in the following manner.

### Experimental Methods

The hippocampus was divided into two parts, one part was lysed with RIPA buffer containing protease inhibitors and the other part was lysed with Trizol reagent (Invitrogen, Rockville, MD, USA) to extract total RNA. The hippocampal RIPA buffer lysate was centrifuged at 5000×g for 10 min and the supernatant was used to measure lipid peroxide and cholesterol content colorimetrically using a lipid peroxidation (MDA) assay kit (Abcam, Cambridge, UK) and a cholesterol kit (Abcam, Cambridge, UK). Supernatants were deproteinized with 1.5 N perchloric acid, and glycogen content was calculated from the glucose concentration derived from glycogen hydrolyzed by α-amyloglucosidase in acid buffer.

Glucose concentration was measured using a glucose kit (Asan Pharmaceutics, Seoul, Korea). Triglycerides were extracted from hippocampus with a chloroform-methanol solution (2:1, v/v) and resuspended in pure chloroform. The triglyceride content in chloroform was determined using a triglyceride colorimetric kit (Asan Pharmaceuticals, Seoul, Korea).

Serum TNF-α and IL-1β concentrations were measured using ELISA kits (eBioscience; San Diego, CA, USA), and serum concentrations of AST and ALT were measured using AST and ALT kits (Asan Pharmaceutics, Seoul, Korea).

### Experimental results

The cholesterol content in hippocampal tissue was significantly higher in the induced group than in the normal control group, while the positive control group and the pork brain enzyme hydrolysate group lowered the cholesterol content.

The glycogen content of hippocampal tissue was higher in the normal control group compared to the induced group, and increased in the low-PBEH (0.053 ml/kg) and medium-PBEH (0.159 ml/kg) groups when pork brain enzyme hydrolysate was treated.

Peroxide in hippocampal tissue was confirmed by MDA content, which was higher in the induced group compared to the normal control group and decreased in the positive control group and the pig brain enzyme hydrolysate-treated group. In particular, the effects of Medium-PBEH (0.159 ml/kg) and Hgh-PBEH (0.478 ml/kg) were as low as the normal control group.

The content of TNF-α, an inflammatory cytokine, in brain tissue was also similar to that of peroxide and was higher in the induced group than in the normal control group, and decreased in the positive control group and in the medium and high PBEH groups.

**[Table 3]**

| | MD-control | Positivel control | Low-PBEH | Medium-PBEH | High-PBEH | Normal-control |
|---|---|---|---|---|---|---|
| Hippocampal triglyceride (mg/g) | 47.5±3.10 | 47.1±1.75 | 47.4±4.36 | 44.7±3.66 | 46.2±3.96 | 46.4±2.45 |
| Hippocampal cholesterol (mg/g) | 18.0±1.21^{a} | 15.0±1.43^{b} | 15.5±1.27^{b} | 13.6±0.76^{c} | 14.5±1.33^{bc} | 12.2±0.82^{d} |
| Hippocampal glycogen (mg/g liver) | 30.5±0.28^{b} | 31.5±0.33^{ab} | 30.9±0.36^{b} | 32.5±0.21^{a} | 32.3±0.42^{a} | 32.7±0.38^{a} |
| Hippocampal lipid peroxides (MDA µmol/g tissue) | 3.85±0.53^{a} | 2.94±0.35^{b} | 3.35±0.54^{a b} | 2.91±0.47^{b} | 2.43±0.44^{c} | 2.31±0.36^{c} |
| Hippocampal TNF-a contents (ng/g tissue) | 4.08±0.26^{a} | 3.41±0.19^{b} | 3.85±0.27^{a b} | 3.43±0.24^{b} | 3.44±0.81^{b} | 3.20±0.11^{c} |
| Acetylcholien esterase activity (U/mg protein) | 0.66±0.09^{a} | 0.32±0.07^{d} | 0.59±0.07^{a b} | 0.53±0.07^{b} | 0.41±0.07^{c} | 0.12±0.05^{e} |
| Relative mRNA expression of hippocampal *TNF-α* (AU) | 1^{a} | 0.88±0.07^{b} | 0.97±0.09^{a} | 0.91±0.09^{ab} | 0.82±0.09^{bc} | 0.78±0.09^{c} |
| Relative mRNA expression of hippocampal *IL-16* (AU) | 1^{a} | 0.94±0.09^{a} | 1.01±0.09^{a} | 0.92±0.09^{ab} | 0.83±0.07^{b} | 0.79±0.08^{b} |
| Relative mRNA expression of hippocampal *BDNF* (AU) | 1^{c} | 1.54±0.19^{a} | 1.07±0.11^{c} | 1.25±0.15^{b} | 1.43±0.11^{a} | 1.36±0.11^{ab} |
| Relative mRNA expression of hippocampal *CNTF* (AU) | 1^{c} | 1.63±0.33^{b} | 1.09±0.18^{c} | 1.39±0.21^{c} | 1.59±0.21^{b} | 3.13±0.64^{a} |

The foregoing description of the invention is for illustrative purposes only, and those having ordinary knowledge in the technical field to which the invention belongs will understand that it can be readily adapted to other specific forms without altering the technical idea or essential features of the invention. It should therefore be understood that the embodiments described above are in all respects exemplary and not limiting.

### [Assignment Information].

Assignment Identification Number: G0210300-01
Assignment Number: 20210210300-0
Ministry: Ministry of Agriculture, Food and Rural Affairs
Research Management Organization: Korea Food Research Institute
Project Name: Food Functionality Evaluation Support Project
Project Title: Study on the Effects and Mechanisms of Porcine Brain Enzyme Hydrolysate on Memory Improvement
Organizer : Unimed Pharmaceutical Co. Kr.
Study period : April 2021 ~ December 2021

## Claims

1. A food composition for improving memory, improving cognitive function, or preventing or improving cerebral neurological disease, comprising a porcine brain enzyme hydrolysate comprising at least one of the peptides consisting of SEQ ID NO: 1 or SEQ ID NO: 2 as an active ingredient.

2. The food composition of claim 1 wherein the porcine brain enzyme hydrolysate is hydrolyzed by one or more proteolytic enzymes.

3. The food composition of claim 1 wherein the peptide is derived from porcine brain hydrolysate.

4. The food composition of claim 1 wherein the porcine brain enzyme hydrolysate exhibits neuroprotective activity and brain-derived neurotrophic factor (BDNF) production activity.

5. The food composition of claim 1 wherein the porcine brain enzyme hydrolysate exhibits acetylcholinesterase inhibitory activity and reactive oxygen species (ROS) inhibitory activity.

6. The food composition of claim 1 wherein the cerebral neurological disease is selected from the group consisting of Alzheimer's Disease, Mild Cognitive Impairment, Senile Dementia, Parkinson's Disease, Huntington's Disease, Lewy Body Dementia, Frontotemporal Dementia, cerebral infarction, stroke, and epilepsy.

7. The food composition of claim 1 wherein the composition is formulated in a dosage form selected from the group consisting of tablet, capsule, powder, granule, liquids, and pill.

8. A pharmaceutical composition for improving memory, improving cognitive function, or preventing or treating cerebral neurological disease, comprising a porcine brain enzyme hydrolysate comprising at least one of the peptides consisting of SEQ ID NO: 1 or SEQ ID NO: 2 as an active ingredient.

9. The pharmaceutical composition of claim 8, wherein the porcine brain enzyme hydrolysate is hydrolyzed by one or more proteolytic enzymes.

10. The pharmaceutical composition of claim 8, wherein the peptide is derived from porcine brain enzyme hydrolysate.

11. The pharmaceutical composition of claim 8, wherein the porcine brain enzyme hydrolysate exhibits neuroprotective activity and brain-derived neurotrophic factor (BDNF) production activity.

12. The pharmaceutical composition of claim 8, wherein the porcine brain enzyme hydrolysate exhibits acetylcholinesterase inhibitory activity and reactive oxygen species (ROS) inhibitory activity.

13. The pharmaceutical composition of claim 8, wherein the cerebral neurological disease is selected from the group consisting of Alzheimer's Disease, Mild Cognitive Impairment, Senile Dementia, Parkinson's Disease, Huntington's Disease, Lewy Body Dementia, Frontotemporal Dementia, cerebral infarction, stroke, and epilepsy.

14. The pharmaceutical composition of claim 8, wherein the composition is formulated in a dosage form selected from the group consisting of tablet, capsule, powder, granule, liquids, and pill.

15. Porcine brain enzyme hydrolysate comprising at least one of the peptides consisting of SEQ ID NO: 1 or SEQ ID NO: 2.

16. The porcine brain enzyme hydrolysate of 15, wherein the peptide is purified or chemically synthesized from a pig brain enzyme hydrolysate.

17. The porcine brain enzyme hydrolysate of 15, wherein the peptide is used to improve memory, improve cognitive ability, or prevent or treat cerebral neurological disease.

18. The porcine brain enzyme hydrolysate of 17, wherein the cerebral neurological disease is selected from the group consisting of Alzheimer's Disease, Mild Cognitive Impairment, Senile Dementia, Parkinson's Disease, Huntington's Disease, Lewy Body Dementia, Frontotemporal Dementia cerebral infarction, stroke, and stroke.

19. The porcine brain enzyme hydrolysate of 15, wherein the peptide exhibits neuroprotective activity and brain-derived neurotrophic factor (BDNF) production activity.

20. The porcine brain enzyme hydrolysate of 15, wherein the peptide exhibits acetylcholinesterase inhibitory activity and reactive oxygen species (ROS) inhibitory activity.

21. Peptide of SEQ ID NO: 1 or SEQ ID NO: 2.

22. The peptide of claim 21, wherein the peptide is purified from porcine brain enzyme hydrolysate or chemically synthesized.

23. The peptide of claim 21, wherein the peptide is used in a food composition or a pharmaceutical composition.

24. The peptide of claim 21, wherein the peptide is used to improve memory, improve cognition, or prevent or treat cerebral neurological disease.

25. The peptide of claim 23, wherein the cerebral neurological disease is selected from the group consisting of Alzheimer's Disease, Mild Cognitive Impairment, Senile Dementia, Parkinson's Disease, Huntington's Disease, Lewy Body Dementia, Frontotemporal Dementia cerebral infarction, stroke, and epilepsy.

26. The peptide of claim 21, wherein the peptide exhibits neuroprotective activity and brain-derived neurotrophic factor (BDNF) production activity.

27. The peptide of claim 21, wherein the peptide exhibits acetylcholinesterase inhibitory activity and reactive oxygen species (ROS) inhibitory activity.

28. A method of improving memory, improving cognitive function, or preventing or treating cerebral neurological disease, comprising the step of administering or taking to an individual a composition comprising a porcine brain enzyme hydrolysate comprising at least one of the peptides consisting of SEQ ID NO: 1 or SEQ ID NO: 2 as an active ingredient.

29. Use of a composition comprising a porcine brain enzyme hydrolysate comprising at least one of the peptides consisting of SEQ ID NO: 1 or SEQ ID NO: 2 as an active ingredient for the manufacture of a medicament/food composition for improving memory, improving cognitive function, or preventing or treating cerebral neurological disease.
